(19) Europäisches Patentamt
European Patent Office
Office européen des brevets

(11) **EP 2 039 815 B1**

(12) **FASCICULE DE BREVET EUROPEEN**

(45) Date de publication et mention
de la délivrance du brevet:
**25.07.2012 Bulletin 2012/30**

(51) Int Cl.:
***D04H 1/02*** *(2006.01)*          ***D04H 1/12*** *(2006.01)*
***D04H 1/46*** *(2012.01)*          ***D04H 13/00*** *(2006.01)*
***A61K 8/02*** *(2006.01)*

(21) Numéro de dépôt: **08290746.0**

(22) Date de dépôt: **01.08.2008**

(54) **Article de nettoyage et/ou de soin de la peau comportant un motif en relief à sa surface et procédé de fabrication dudit article**

Gegenstand zur Reinigung und/oder Pflege der Haut, der ein Reliefmotiv an seiner Oberfläche umfasst und Herstellungsverfahren besagten Gegenstands

Cleaning and/or skin care item comprising a raised pattern on its surface and production method thereof

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **17.08.2007 FR 0705878**

(43) Date de publication de la demande:
**25.03.2009 Bulletin 2009/13**

(73) Titulaire: **Georgia-Pacific France**
**92270 Bois-Colombes (FR)**

(72) Inventeurs:
• **Bret, Bruno**
**68920 Wintzenheim (FR)**

• **Clermont, Anne-Gaëlle**
**68000 Colmar (FR)**
• **Gregoire, Philippe**
**27700 Les Andelys (FR)**
• **Louis dit Picard, Bernard**
**27370 Amfreville la Campagne (FR)**

(74) Mandataire: **Cortier, Sophie et al**
**Georgia-Pacific France**
**Service Propriété Industrielle**
**60, avenue de l'Europe**
**92270 Bois-Colombes (FR)**

(56) Documents cités:
**EP-A- 0 819 393          EP-A- 1 106 723**
**EP-A- 1 310 226          WO-A-94/17235**
**US-A1- 2007 098 768**

**Description**

L'invention concerne un article de nettoyage et/ou de soin de la peau, tel qu'un tampon à démaquiller à base de fibres de coton hydrophiles, destiné à appliquer et/ou à enlever des produits liquides ou semi-solides sur la peau.

La plupart des articles de nettoyage et/ou de soin de la peau présents sur le marché se présentent sous la forme de formats découpés, ronds, ovales ou carrés. Ils sont souvent composés d'un mélange de fibres de coton de différentes qualités ou d'un mélange de fibres de coton et d'autres fibres selon les caractéristiques d'absorption ou de résistance désirées. Néanmoins, il est également envisageable d'utiliser des matières non tissées formées uniquement à partir de fibres synthétiques ou artificielles.

Quel que soit le type de fibres utilisées, on constate que ies articles de nettoyage et/ou de soin du commerce possèdent une composition sensiblement homogène dans toute leur épaisseur. Il en résulte une relative symétrie entre chacune des faces externes desdits articles.

Tel qu'il est décrit dans la demande de brevet international WO 01/42548 déposée au nom de la demanderesse, cette absence de différentiation entre les deux faces externes de l'article ne permet pas de leur affecter à l'avance un usage ou une fonction particulière dans l'opération de nettoyage et de soin de la peau. Dans la demande de brevet susmentionnée, au contraire, on envisage de former un tampon à démaquiller en coton possédant deux faces distinctes, l'une destinée aux soins de la peau, en particulier au nettoyage de la peau et à l'application de produits cosmétiques de maquillage ou de démaquillage, et l'autre plus douce et plus absorbante, destinée à absorber l'excédent de produit appliqué.

La différentiation se faisant principalement par la présence de motifs en creux de type stries sur l'une des faces externes du tampon, l'utilisateur est capable de distinguer les faces soit au toucher, soit visuellement.

Dans cet art antérieur, on avait également constaté que la présence d'un relief, notamment de type stries, favorisait l'efficacité au démaquillage.

Tirant partie de cette dernière constatation, la demanderesse a cherché à renforcer encore le relief présent à la surface desdits tampons.

En particulier, elle a cherché à obtenir des tampons à démaquiller, et plus généralement des articles de nettoyage et/ou de soin de la peau, possédant un relief prononcé, notamment à l'état humide.

En effet, jusqu'à présent, le relief formé à la surface des tampons résulte généralement d'une compression des fibres soit sous l'effet d'un gaufrage ou d'un calandrage, soit sous l'effet d'un procédé d'hydroliage destiné à lier plusieurs nappes de fibres ensemble.

L'inconvénient dans ces méthodes de formation de relief est la difficulté à obtenir des creux ou des reliefs de grande amplitude. L'obtention de tels reliefs s'accompagne notamment d'un risque accru de perforation ou de déchirure de la nappe de fibres ainsi déformée et, de ce fait, entraîne une fragilisation du tampon au final, laquelle n'est pas acceptable dans le cadre de l'utilisation cosmétique envisagée.

Par ailleurs, une fois humidifié, le relief perd souvent de son amplitude, les fibres du tampon ayant tendance à reprendre leur positionnement initial dans la nappe.

L'invention vise donc à fournir un article de nettoyage et/ou de soin de la peau ne présentant pas les inconvénients de l'art antérieur.

Elle vise en particulier à fournir un article de nettoyage et/ou de soin de la peau présentant un relief prononcé, même à l'état humide, sur au moins l'une de ses faces externes, l'article possédant par ailleurs une bonne résistance et une bonne cohésion de façon à éviter tout risque de déchirure ou de perforation lors de son utilisation.

A cet effet, et conformément à l'invention, il est proposé un article de nettoyage et/ou de soin de la peau, tel qu'un tampon à démaquiller à base de fibres de coton hydrophiles, destiné à appliquer et/ou à enlever des substances liquides ou semi-solides sur la peau, comprenant au moins deux couches externes en matière fibreuse absorbante liées ensemble et au moins une série de fils disposée entre lesdites couches externes, l'épaisseur d'au moins une des couches externes étant inférieure au diamètre moyen des fils de manière à créer un motif en relief à la surface de l'article.

Selon une configuration particulière de l'invention, la liaison des couches externes est réalisée au moyen d'une technique choisie parmi l'hydroliage, le collage et la thermofusion.

Selon une configuration particulière de l'invention, l'article comprend deux séries de fils disposées entre les couches externes, les fils de chacune des séries étant sensiblement parallèles entre eux et formant un angle $\alpha$ avec les fils de l'autre série.

Selon une autre configuration particulière de l'invention, les deux séries de fils sont solidaires au sein d'une unique grille tramée.

Selon une autre configuration particulière de l'invention, l'angle $\alpha$ est sensiblement égal à 90°.

Selon une autre configuration particulière de l'invention, le motif en relief possède des protubérances d'une hauteur H comprise entre 0,2 et 2,0 mm, et de préférence comprise entre 0,3 et 0,5 mm.

L'invention a également pour objet un article de nettoyage et/ou de soin de la peau, tel qu'un tampon à démaquiller à base de fibres de coton hydrophiles, destiné à appliquer et/ou à enlever des substances liquides ou semi-solides sur la peau, comprenant au moins deux couches externes (10a, 10b) en matière fibreuse absorbante liées ensemble et au moins une série de fils (10c, 10d, 10e, 10f) disposée entre lesdites couches externes (10a, 10b) caractérisé en ce que ladite série de fils crée un motif en relief à la surface de l'article, ledit motif possédant des protubérances (5) d'une hauteur H et en ce que le rapport Rh entre la hauteur H des protubérances (5) du motif en relief lorsque l'article est humide et la hauteur H des protubérances (5) du motif en relief lorsque l'article est sec est supérieur à 0,7 et de préférence supérieur à 1.

Selon une autre configuration particulière de l'invention, le motif en relief définit une pluralité de cavités destinées à recevoir le produit de nettoyage et/ou de soin.

Selon une autre configuration particulière de l'invention, l'article possède une résistance à la traction à l'état sec d'au moins 35 N en sens marche et d'au moins 20 N en sens travers, et de préférence supérieure à 50 N en sens marche et en sens travers, selon la méthode de test donnée dans la description.

Selon une autre configuration particulière de l'invention, l'article possède un indice de friction moyen à l'état sec et à l'état humide supérieur à 0,35 et, en particulier, un indice de friction moyen à l'état humide supérieur à l'indice de friction moyen à l'état sec.

Ainsi configurée, l'invention est susceptible de fournir un article de nettoyage et/ou de soin de la peau possédant un motif en relief à sa surface lequel est formé non pas par compression des nappes de fibres, mais par la présence sous les couches externes de l'article d'une série de fils de gros diamètre.

Par fils, il faut entendre toute structure solide se présentant sous une forme effilée. Ceci regroupe donc les fils obtenus par un procédé de filature ou un procédé d'extrusion et formés à partir de matière naturelle, comme le coton, la soie, le lin, artificielle, comme la viscose, synthétique, comme le polypropylène, minérale, comme le verre, métallique, comme l'acier ou l'aluminium.

Ceci regroupe également les parties effilées de certaines structures bidimensionnelles, comme les grilles extrudées ou moulées ou les trames textiles telles que le tissu.

La déformation de la ou des couches externes sera d'autant plus importante que le diamètre des fils sera grand. En outre, cette déformation dépendra également du nombre de fils présents sous la ou les couches externes et de leur disposition les uns par rapport aux autres. Ainsi, deux fils superposés produiront une déformation deux fois plus importante.

Ce motif en relief sera également beaucoup plus stable lors d'une humidification de l'article, étant donné qu'aucune réorganisation des fibres de la nappe ne pourra se produire dans les zones recouvrant le fil.

Par ailleurs, du fait de la rigidité des fils utilisés, la résistance et la cohésion de l'article sera également renforcée.

L'invention a également pour objet un procédé de fabrication d'un article de nettoyage et/ou de soin de la peau, comportant les étapes suivantes :

- formation d'au moins une première couche externe dudit article à partir d'une nappe de fibres absorbantes,
- formation d'au moins une deuxième couche externe dudit article à partir d'une nappe de fibres absorbantes,
- insertion d'au moins une première série de fils entre lesdites première et deuxième couches externes,
- assemblage desdites couches externes et de ladite série de fils.

Selon une configuration particulière de l'invention, l'assemblage des couches externes et de la série de fils s'effectue au moyen d'une technique choisie parmi l'hydroliage, le collage et la thermofusion.

Selon une autre configuration particulière de l'invention, on insère une deuxième série de fils entre les première et deuxième couches externes, les fils de chacune des première et deuxième séries étant sensiblement parallèles entre eux et formant un angle $\alpha$ avec les fils de l'autre série.

Selon une autre configuration particulière de l'invention, les fils de la première série sont déposés après et au-dessus des fils de la deuxième série.

Selon une autre configuration particulière de l'invention, les fils de ia deuxième série sont déposés de manière à former avec les fils de la première série une structure semblable à une trame textile, les fils de la première série formant les fils de trame et les fils de la deuxième série formant les fils de chaîne.

Selon une autre configuration particulière de l'invention, l'angle $\alpha$ est sensiblement égal à 90°.

Selon une autre configuration particulière de l'invention, les nappes de fibres sont formées essentiellement à partir de fibres de coton hydrophiles.

Selon une autre configuration particulière de l'invention, les nappes de fibres comprennent de 70 à 100 % de fibres de coton et de 0 à 30 % de fibres artificielles, choisies notamment parmi les fibres de viscose, de fibres synthétiques, telles que des fibres de polyester, des fibres bicomposantes du type polyester/polyester, polypropylène/polypropylène ou polyester/ polypropylène, ou leurs mélanges.

Selon une autre configuration particulière de l'invention, les fils sont fabriqués à partir d'un matériau choisi parmi les polymères d'origine naturelle, artificielle, synthétique, les matières métalliques et les matières minérales.

Selon une autre configuration particulière de l'invention, les fils sont formés selon un procédé choisi parmi la filature, l'extrusion et le moulage.

L'invention vise également un article de nettoyage et/ou de soin de la peau, tel qu'un tampon à démaquiller à base de fibres de coton hydrophiles, destiné à appliquer et/ou à enlever des substances liquides ou semi-solides sur la peau, comprenant au moins une première couche externe en matière fibreuse absorbante et au moins une seconde couche externe comprenant une série de fils, lesdits fils formant un motif en relief à la surface de l'article.

D'autres caractéristiques et avantages de l'invention apparaîtront plus en détails dans la description qui suit et en référence aux dessins annexés dans lesquels :

- la figure 1 représente une vue de profil d'un article de nettoyage et/ou de soin selon l'invention,
- les figures 2A, 2B, 2C et 2D représentent différents motifs en relief pouvant être appliqués sur un article conforme à l'invention,
- les figures 3A, 3B, 3C et 3D représentent différents modes de fabrication d'un article conforme à l'invention,
- les figures 4A, 4B, 4C, 4D, 4E, 4F et 4G sont les photographies des faces recto et verso des tampons à démaquiller cités dans les exemples.

Un article de nettoyage et/ou de soin de la peau conforme à l'invention est généralement un produit découpé de forme ronde, ovale, carrée ou de tout autre forme. Il a un grammage compris entre 80 et 400 g/m$^2$ et de préférence compris entre 100 et 300 g/m$^2$. Il peut être formé de fibres de coton hydrophiles absorbantes. En particulier, il peut comprendre de 70 à 100 pour cent de fibres de qualité homogène et de 0 à 30 pour cent de fibres artificielles telles que des fibres de viscose, et/ou de fibres synthétiques telles que des fibres de polyester, des fibres bicomposantes (polyester/polyester, polypropylène/polypropylène ou polyester/polypropylène), ou leurs mélanges. Il est bien entendu envisageable d'utiliser tout autre type de fibres naturelles, synthétiques ou artificielles et selon des proportions différentes pour fabriquer ledit article : le choix du type de fibres et des proportions se fera dans ce cas de manière à conférer à l'article les caractéristiques d'absorption et de douceur recherchées au final.

Dans tous les exemples qui suivent, l'article sera formé à partir d'un même support de base, à savoir une nappe épaisse, mono ou multicouches, formée de fibres de coton sur laquelle on a déposé une nappe mince, du type voile de carde, formée également de fibres de coton, l'ensemble étant hydrolié de manière à lier les nappes entre elles. La nappe épaisse aura par exemple un grammage compris entre 100 et 250 g/m$^2$ et la nappe mince aura un grammage compris entre 10 et 80 g/m$^2$.

Avant l'étape d'hydroliage, on aura au préalable inséré une ou plusieurs séries de fils. Ces fils auront un diamètre supérieur à l'épaisseur de la nappe mince de manière à la déformer suffisamment et créer ainsi un motif en relief à la surface de l'article. Il est bien entendu envisageable d'accentuer ce relief en superposant une ou plusieurs séries de fils. L'épaisseur de la nappe mince devra dans ce cas être inférieure à la somme des diamètres de chacun des fils superposés. Le diamètre des fils pourra notamment être compris entre 200 et 2000 micromètres, et de préférence compris entre 300 et 800 micromètres. Les fils pourront être formés en un polymère naturel, artificielle ou synthétique ou en une matière minérale ou métallique. De préférence, pour former les fils, on utilisera une matière gonflant à l'humidité, comme le coton, de manière à créer un relief variable selon les conditions d'humidité de l'article, en particulier un relief plus accentué lorsque l'article est humide (c'est-à-dire lorsqu'il contient plus de 100 % en poids d'eau). Ce gonflement sera variable en fonction du degré de torsion du fil. En effet, plus le fil sera torsadé sur lui-même, moins il aura tendance à gonfler sous l'effet de l'humidité.

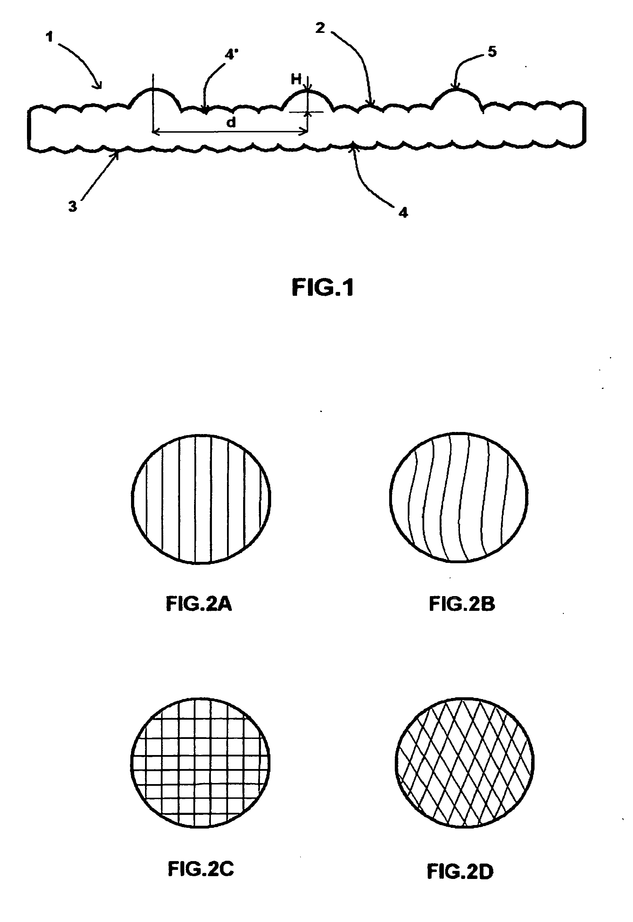

En référence à la figure 1, il est représenté une vue schématique de profil d'un article de nettoyage et/ou de soin de la peau conforme à l'invention.

L'article, ou tampon 1, comprend une première face externe 2 et une seconde face externe 3. La première et la seconde face externe 2 et 3 présentent des stries 4 et 4' disposées parallèlement les unes aux autres. Les stries sont formées par les jets d'eau sortant des buses du dispositif d'hydroliage, l'écartement et la puissance des jets d'eau déterminant respectivement l'écartement et la profondeur des stries 4 et 4'. Ainsi, l'écartement des stries 4 et 4' pourra être compris entre 0,4 et 1,2 mm et la profondeur des stries 4 pourra être inférieure à 0,25 mm, et en particulier de l'ordre de 0,1 mm.

Le tampon 1 comporte par ailleurs une série de protubérances 5, sensiblement semi cylindriques, sur sa surface externe 2. Comme vu précédemment, ces protubérances 5 sont formées sur les zones de la nappe mince de fibres de coton recouvrant les fils textiles rigides. Ces protubérances pourront avoir une hauteur H comprise entre 0,2 mm et 2,0 mm, et de préférence comprise entre 0,3 mm et 0,5 mm. La distance d séparant chacune des protubérances 5 pourra varier en fonction du motif en relief à former et en fonction de la zone du motif observée. Dans un souci d'efficacité au démaquillage, on privilégiera toutefois les motifs en relief pour lesquels la distance d entre chaque protubérance 5 est sensiblement égale sur toute la largeur du tampon 1 et est comprise entre 2 et 15 mm et de préférence entre 5 et 12 mm. Il est toutefois envisageable de faire varier cette distance d sur certaines zones du motif en relief de manière à créer des zones plus ou moins rugueuses sur le tampon 1 et ainsi modifier localement l'efficacité au démaquillage dudit tampon 1.

En référence aux figures 2A à 2D, il est représenté plusieurs exemples de motifs en relief formés à la surface d'un article de nettoyage et/ou de soin conforme à l'invention. Ainsi, les protubérances pourront former à la surface de l'article des lignes continues droites (figure 1, figure 2A) ou courbes (figure 2B). Elles pourront également former un grille à mailles carrées (figure 2C) ou en losanges (figure 2D).

Par ailleurs, de manière à faciliter l'application de produits cosmétiques sur l'article et par la suite le transfert de ces produits sur la peau, il sera avantageux de former des cavités réceptrices à la surface de l'article. Ces cavités réceptrices seront délimitées par les protubérances du motif en relief. Ainsi, sur les figures 2C, respectivement 2D, chaque carré, respectivement losange, du motif en relief pourra potentiellement former une cavité réceptrice pour les produits de nettoyage et/ou de soin.

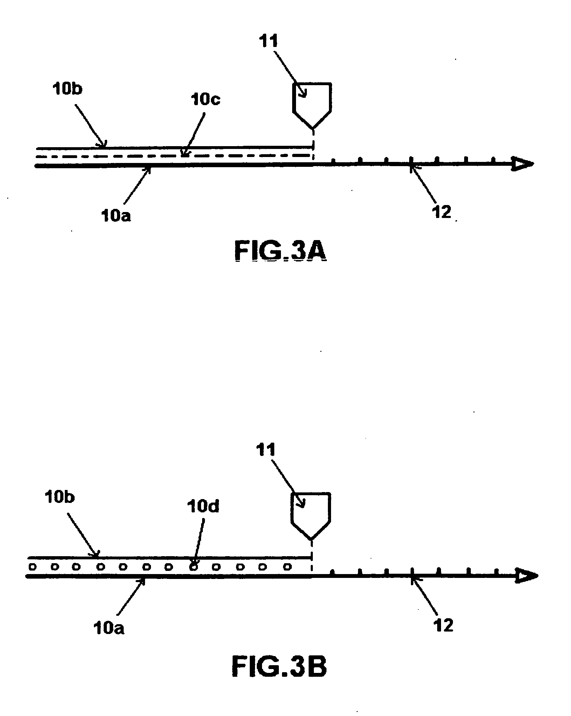

En référence aux figures 3A à 3D, il est représenté diverses variantes de réalisation de l'article selon l'invention.

Quel que soit la variante envisagée, on réalise toujours le même support de base pour l'article, tel que défini précédemment, en réalisant d'une part une nappe fibreuse épaisse 10a, d'un grammage compris entre 100 et 250 $g/m^2$, et d'autre part une nappe fibreuse mince 10b, d'un grammage compris entre 10 et 80 $g/m^2$. La superposition des deux nappes est ensuite hydroliée au moyen d'un dispositif d'hydroliage 11. Le procédé de formation d'un tel support de base a notamment été décrit en détail dans le brevet européen EP 0 681 621 déposée au nom de la demanderesse.

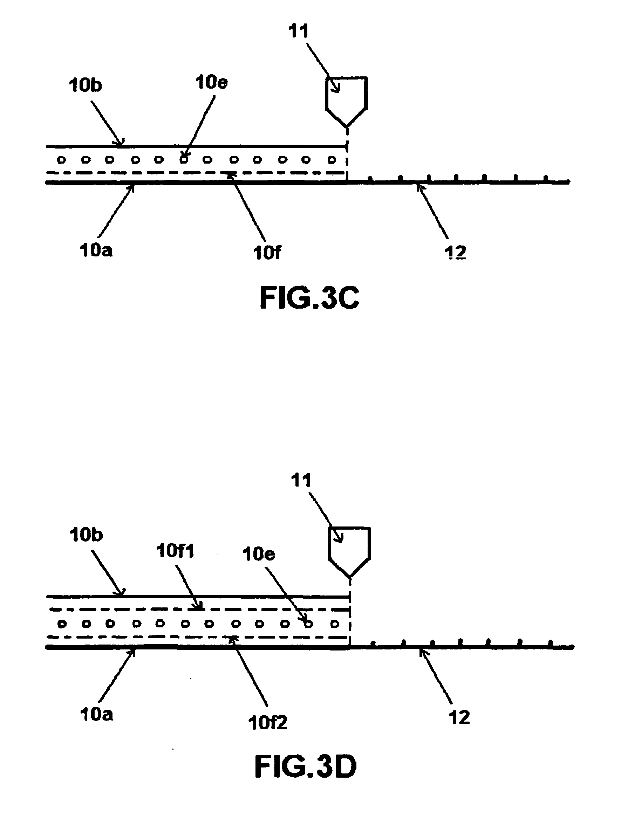

Préalablement à l'étape d'hydroliage, et en fonction de la variante envisagée, on pourra insérer entre la nappe épaisse et la nappe mince soit une grille tissée 10c déjà formée (figure 3A), soit une série de fils textiles 10d parallèles entre eux (figure 3B), soit une première série de fils textiles 10e parallèles entre eux et une deuxième série de fils textiles 10f parallèles entre eux, les fils 10e de la première série étant superposés et sensiblement orthogonaux aux fils 10f de la deuxième série (figure 3C), soit une première série de fils textiles 10e parallèles entre eux et une deuxième série de fils textiles 10f1 et 10f2 parallèles entre eux, chaque fil 10e de la première série étant disposé alternativement au-dessous d'un fil 10f1 et au-dessus d'un fil 10f2 de la deuxième série, à la manière d'un fil de trame maintenu entre les fils de chaîne d'un matériau textile, les fils 10e de la première série étant sensiblement orthogonaux aux fils 10f1 et 10f2 de la deuxième série (figure 3D).

L'insertion des fils ou de la grille tissée entre les deux nappes de fibres 10a et 10b pourra s'effectuer notamment au moyen d'une navette, d'une lance, d'un jet d'air ou d'un jet d'eau, cette liste n'étant pas limitative.

Une fois l'hydroliage de l'ensemble effectué, on obtient donc une nappe liée 12 présentant une série de protubérances sur l'une au moins de ses surfaces externes, laquelle forme un motif en relief.

La nappe liée 12 est ensuite découpée au format des articles à obtenir.

TESTS COMPARATIFS :

On a effectué une série de mesures sur des produits conformes à l'invention et sur des produits du commerce. Les produits utilisés à ce niveau sont respectivement :

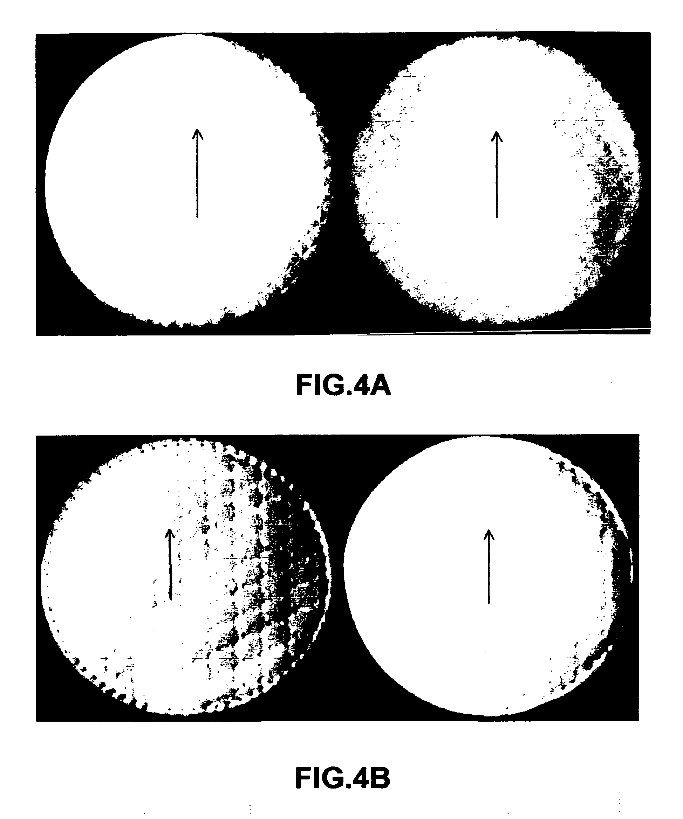

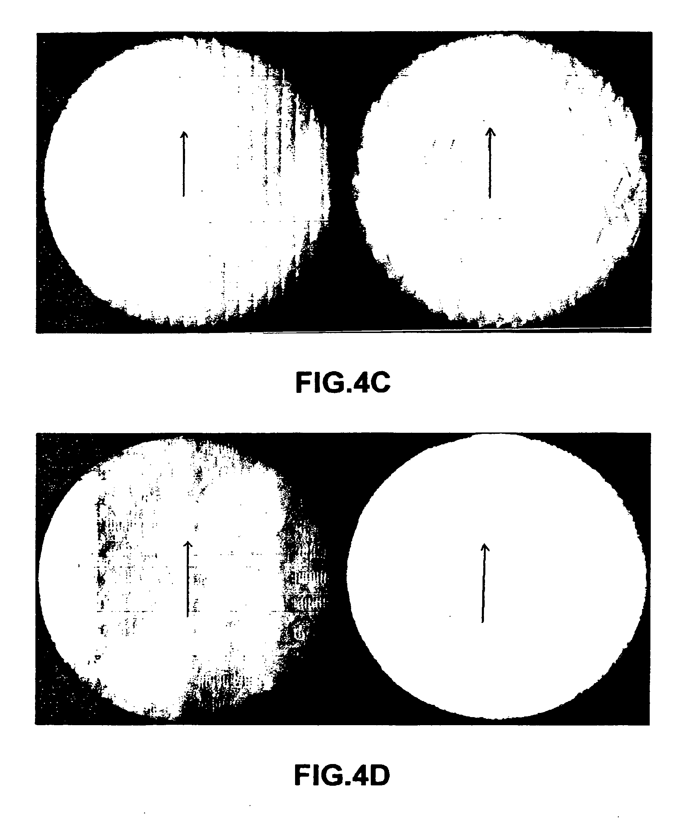

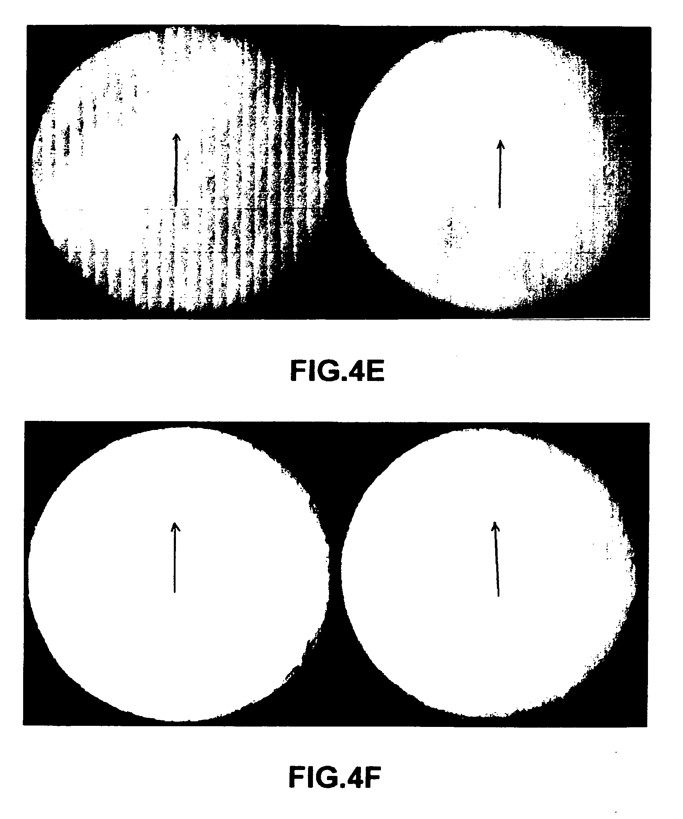

- produit A: tampon à démaquiller de marque AUCHAN formé par l'assemblage par thermofusion de fibres de coton et de fibres thermofusibles, puis formation d'un relief par calandrage sur la face recto (figure 4A) ;
- produit B : tampon à démaquiller de marque SWISSPERS obtenu par formation d'un relief par calandrage sur ses faces recto et verso et assemblage par poinçonnage périphérique de trois nappes de coton (figure 4B) ;
- produit C: tampon à démaquiller de marque CORA formé par l'assemblage par un liant de trois nappes de coton, les deux nappes externes ayant été au préalable calandrées individuellement (figure 4C) ;
- produit D : tampon à démaquiller de marque CHAMPION formé par hydroliage simple d'une nappe de coton sur sa face verso, et double hydroliage sur sa face recto (figure 4D);
- produit E : tampon à démaquiller de marque Demak'up et conforme à la demande de brevet internationale WO 01/42548 (figure 4E);
- produit F : tampon à démaquiller conforme à l'invention, comportant trois séries de fils de coton insérées entre deux nappes de coton, les fils formant un motif en relief de type grille à la surface du tampon, un fil de gros diamètre, constituant le fil de trame, étant emprisonné entre deux fils de petit diamètre, constituant le fil de chaîne (figure 4F); les caractéristiques des fils de coton utilisées sont :

  - diamètre du fil de trame (état sec) = 560 $\mu$m
  - diamètre du fil de chaîne (état sec) = 310 $\mu$m
  - diamètre du fil de trame (état humide) = 670 $\mu$m
  - diamètre du fil de chaîne (état humide) = 390 $\mu$m

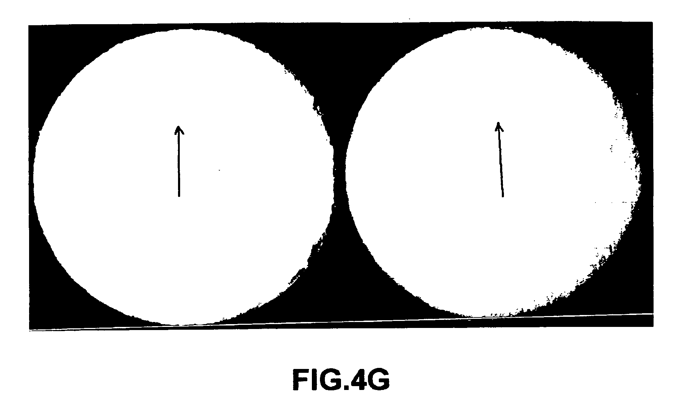

- produit G : tampon à démaquiller conforme à l'invention (figure 4G), se différentiant du produit F par le diamètre des fils utilisés :

- diamètre du fil de trame (état sec) = 580 $\mu$m
- diamètre du fil de chaîne (état sec) = 330 $\mu$m
- diamètre du fil de trame (état humide) = 710 $\mu$m
- diamètre du fil de chaîne (état humide) = 410 $\mu$m

Les mesures effectuées sur ces produits sont respectivement :

- le grammage W (en g/m$^2$)
- l'épaisseur e (en mm)
- la résistance à la traction RT (en N) à l'état sec, dans le sens marche et dans le sens travers
- la hauteur H des protubérances (en micromètres) à l'état sec et à l'état humide, dans le sens marche et dans le sens travers
- l'indice de friction IF à l'état sec et à l'état humide, dans le sens marche et dans le sens travers

Pour les tests donnés ci-dessous, l'état sec du produit correspond à l'état initial du produit, non imprégné, et l'état humide du produit correspond à un état où le produit a été imprégné avec environ 800 % en poids d'eau distillée.
De manière à uniformiser la répartition de l'eau dans le produit à l'état humide, on pose un poids de 3540 g pendant 60 secondes sur le produit juste après son imprégnation avec l'eau distillée.
En outre, on a indiqué sur les figures 4A à 4G, au moyen d'une flèche, le sens marche tel qu'il a été mesuré sur chacun des produits et sur chacune des faces.
Le sens travers correspond évidemment à la direction qui est orthogonale au sens marche.
Chacune des méthodes de mesures utilisées lors de ces test comparatifs est explicitée ci-dessous.

MESURE DE L'EPAISSEUR DES TAMPONS :

On pose un poids de 53,7 g sur une pile de 20 tampons à démaquiller.
On mesure la hauteur de la pile ainsi comprimée.
On divise la hauteur mesurée par 20.
La valeur ainsi calculée correspond à l'épaisseur e.

MESURE DE L'EPAISSEUR DE LA COUCHE EXTERNE :

Préalablement à la formation du tampon, on colore à saturation les fils avec du bleu COVANOR W 6605 commercialisé par la société LCW.
Après la formation du tampon, on verse de l'azote liquide sur le tampon ainsi formé de manière à recouvrir le tampon pendant 30 secondes puis, sans toucher les bords, on effectue une découpe transversale du tampon de manière à faire apparaître distinctement sur la tranche du tampon la succession des couches externes de fibres et des couches internes de fils du tampon.
On replonge rapidement l'échantillon dans l'azote liquide puis on laisse sécher à l'air libre.
Une fois que l'azote est totalement évaporé, on place l'échantillon sur un support plan de manière à pouvoir observer la tranche.
Au moyen d'une loupe binoculaire avec un grossissement de 40X et à l'aide d'une mire graduée, installée sur la loupe binoculaire, on fait des mesures de l'épaisseur de la couche externe qui recouvrent les fils tintés en bleu.
Les mesures effectuées sur les tampons F et G conformes à l'invention donnent une épaisseur moyenne pour la couche externe d'environ 150 $\mu$m, laquelle est très inférieure au diamètre moyen des fils utilisés.

MESURE DE LA RESISTANCE A LA TRACTION :

Des échantillons ou éprouvettes de 57 mm de long sur 25 mm de large sont découpés dans les tampons à tester. Une première série d'échantillons est découpée de manière à obtenir la plus grande longueur de l'échantillon dans le sens marche et de manière à couvrir les reliefs de la surface. Une seconde série d'échantillons est découpée de manière à obtenir la plus grande longueur de l'échantillon dans le sens travers et de manière à couvrir également les reliefs de la surface.
La mesure de la résistance est effectuée au moyen d'un dynamomètre. On place l'échantillon entre les deux mâchoires du dynamomètre écartées de 30 mm dans le sens de la longueur de l'échantillon. On écarte les mâchoires à une vitesse de 100 mm/min et on mesure la force maximale exercée avant rupture.
Cette force maximale est la résistance à la traction RT.

MESURE DE LA HAUTEUR DES PROTUBÉRANCES :

On dépose un tampon à démaquiller non imprégné sous une caméra vidéo numérique reliée à un ordinateur, la face du tampon comportant le motif en relief étant tournée vers la caméra.

On traite les données exportées au moyen d'un logiciel d'analyse de prise de vue tridimensionnelle par projection de franges et au moyen d'un logiciel de mesure TOPOSURF.

Ces logiciels permettent de donner une topographie de surface très précise des régions observées.

On sélectionne une série de 3 régions homogènes de mesure dans le sens marche et dans le sens travers, de préférence sur les zones du tampon comportant les hauteurs de protubérances les plus élevées.

On mesure la hauteur H des protubérances dans ces régions.

On fait la moyenne des 3 mesures effectuées : on obtient la hauteur H des protubérances pour l'une des faces du tampon dans le sens marche et dans le sens travers.

On réalise ensuite une série de mesures similaires lorsque le tampon est dans son état humide.

On calcule ensuite le rapport Rh :

$$Rh = (\text{hauteur H mesurée à l'état humide}) / (\text{hauteur H mesurée à l'état sec}).$$

MESURE DE L'INDICE DE FRICTION :

On utilise un appareil de test de friction fourni par la société KATO Tech Co Ltd sous la référence Friction Tester.

Cet appareil fonctionne selon la méthode KAWABATA et fournit au final un indice IF, dit indice de friction de l'échantillon.

Plus l'indice IF est élevé, plus la face testée de l'échantillon est rugueuse.

On positionne les échantillons à tester de manière à orienter les faces munies du relief vers la cellule de mesure.

On effectue ainsi 4 mesures : selon le sens marche et le sens travers, à l'état sec et à l'état humide.

On calcule ensuite l'indice de friction moyen (IFM) pour l'échantillon sec et humide :

$$IFM = (IFsm \times IFst)^{1/2},$$

avec IFsm = indice de friction dans le sens marche

et IFst = indice de friction dans le sens travers.

Les résultats ont été regroupés dans les tableaux 1 à 3.

TABLEAU 1

| produit | W | e | RT Sens marche | RT Sens travers |
|---------|-----|----|----------------|-----------------|
| A | 193 | 48 | 9 | 9 |
| B | 249 | 56 | 7 | 2 |
| C | 288 | 78 | 5 | 4 |
| D | 232 | 56 | 7 | 5 |
| E | 235 | 60 | 21 | 14 |
| F | 219 | 50 | 75 | 66 |
| G | 233 | 50 | 71 | 67 |

TABLEAU 2

| Produit | H sec sens marche | H hum sens marche | Rh sens marche | H sec sens travers | H hum sens travers | Rh sens travers |
|---------|-------------------|-------------------|----------------|--------------------|--------------------|-----------------|
| A | 286 | 284 | 0,99 | 279 | 277 | 0,99 |

(suite)

| Produit | H sec sens marche | H hum sens marche | Rh sens marche | H sec sens travers | H hum sens travers | Rh sens travers |
|---|---|---|---|---|---|---|
| B | 470 | 284 | 0,60 | 455 | 243 | 0,53 |
| C face ligne | | | | 260 | 160 | 0,62 |
| C face chevron | 252 | 130 | 0,52 | 219 | 120 | 0,55 |
| D | 388 | 258 | 0,66 | 398 | 270 | 0,68 |
| E | | | | 230 | 150 | 0,65 |
| F | 300 | 424 | 1,41 | 353 | 220 | 0,62 |
| G | 273 | 405 | 1,48 | 335 | 257 | 0,77 |

TABLEAU 3

| Produit | IF sec sens marche | IF sec sens travers | IFM sec | IF hum sens marche | IF hum sens travers | IFM hum |
|---|---|---|---|---|---|---|
| A | 0,20 | 0,19 | 0,19 | 0,18 | 0,19 | 0,18 |
| B | 0,21 | 0,23 | 0,22 | 0,22 | 0,20 | 0,21 |
| C face ligne | 0,22 | 0,23 | 0,22 | 0,20 | 0,22 | 0,21 |
| C face chevron | 0,24 | 0,43 | 0,32 | 0,22 | 0,26 | 0,24 |
| D | 0,21 | 0,25 | 0,23 | 0,21 | 0,20 | 0,20 |
| E | 0,20 | 0,23 | 0,21 | 0,17 | 0,19 | 0,18 |
| F | 0,31 | 0,41 | 0,36 | 0,30 | 0,56 | 0,41 |
| G | 0,39 | 0,48 | 0,43 | 0,47 | 0,55 | 0,51 |

On constate donc que les tampons conformes à l'invention F et G possèdent une résistance à la traction largement supérieure à celle des tampons du commerce, et notamment supérieure à 65 N.

On constate également que les hauteurs de protubérances mesurées sur les tampons conformes à l'invention sont supérieures aux hauteurs mesurées sur les tampons du commerce dans le sens marche et à l'état humide.

Par ailleurs, on constate de façon surprenante que cette hauteur augmente à l'état humide et dans le sens marche pour les tampons conformes à l'invention par rapport à celle mesurée à l'état sec (Rh>1).

Ceci peut s'expliquer par la capacité des fils de coton insérés entre les nappes de fibres à gonfler sous l'effet de l'humidité.

Cette capacité à augmenter de volume, et donc de diamètre, ne se constate toutefois que pour certains fils de coton, étant donné les résultats observés dans le sens travers (RH<1).

Sans être tenue par une quelconque théorie, il est probable que plus le fil de coton sera torsadé, moins il aura la capacité d'augmenter de diamètre à l'état humide.

On constate finalement que l'indice de friction moyen IFM des tampons conformes à l'invention est supérieur à celui des tampons du commerce, que ce soit à l'état sec ou à l'état humide.

En particulier, on constate que, pour les tampons conformes à l'invention, l'indice de friction moyen (IFM) est supérieur à 0,35, que ce soit à l'état sec et à l'état humide, et l'indice de friction moyen à l'état humide est supérieur à l'indice de friction moyen à l'état sec.

Les tampons de l'invention seront donc plus rugueux que ceux du commerce et auront donc une efficacité de démaquillage améliorée, notamment à l'état humide.

**Revendications**

1. Article (1) de nettoyage et/ou de soin de la peau, tel qu'un tampon à démaquiller à base de fibres de coton hydrophiles, destiné à appliquer et/ou à enlever des substances liquides ou semi-solides sur la peau, comprenant au moins deux couches externes (10a,10b) en matière fibreuse absorbante liées ensemble et au moins une série de fils (10c,10d, 10e,10f) disposée entre lesdites couches externes (10a,10b) **caractérisé en ce que** l'épaisseur d'au moins une des couches externes (10b) est inférieure au diamètre moyen des fils de manière à créer un motif en relief à la surface de l'article.

2. Article (1) selon la revendication 1, **caractérisé en ce qu'**il comprend deux séries de fils (10e,10f) disposées entre les couches externes (10a,10b), les fils de chacune des séries étant sensiblement parallèles entre eux et formant un angle α avec les fils de l'autre série.

3. Article (1) selon la revendication 2, **caractérisé en ce que** les deux séries de fils sont solidaires au sein d'une unique grille tramée (10c).

4. Article (1) selon l'une quelconque des revendications 2 ou 3, **caractérisé en ce que** l'angle α est sensiblement égal à 90°.

5. Article (1) selon l'une quelconque des revendications précédentes, **caractérisé en ce que** le motif en relief possède des protubérances (5) d'une hauteur H comprise entre 0,2 et 2,0 mm, et de préférence comprise entre 0,3 et 0,5 mm.

6. Article (1) selon l'une quelconque des revendications précédentes, **caractérisé en ce que** le rapport Rh entre la hauteur H des protubérances (5) du motif en relief lorsque l'article est humide et la hauteur H des protubérances (5) du motif en relief lorsque l'article est sec est supérieur à 0,7, et de préférence supérieur à 1.

7. Article (1) de nettoyage et/ou de soin de la peau, tel qu'un tampon à démaquiller à base de fibres de coton hydrophiles, destiné à appliquer et/ou à enlever des substances liquides ou semi-solides sur la peau, comprenant au moins deux couches externes (10a, 10b) en matière fibreuse absorbante liées ensemble et au moins une série de fils (10c, 10d, 10e, 10f) disposée entre lesdites couches externes (10a, 10b) **caractérisé en ce que** ladite série de fils crée un motif en relief à la surface de l'article, ledit motif possédant des protubérances (5) d'une hauteur H et **en ce que** le rapport Rh entre la hauteur H des protubérances (5) du motif en relief lorsque l'article est humide et la hauteur H des protubérances (5) du motif en relief lorsque l'article est sec est supérieur à 0,7 et de préférence supérieur à 1.

8. Article (1) selon l'une quelconque des revendications précédentes, **caractérisé en ce que** le motif en relief définit une pluralité de cavités destinées à recevoir le produit de nettoyage et/ou de soin.

9. Article (1) selon l'une quelconque des revendications précédentes, **caractérisé en ce qu'**il possède une résistance à la traction à l'état sec d'au moins 35 N en sens marche et d'au moins 20 N en sens travers, et de préférence supérieure à 50 N en sens marche et en sens travers, selon la méthode de test donnée dans la description.

10. Article (1) selon l'une quelconque des revendications précédentes, **caractérisé en ce qu'**il possède un indice de friction moyen à l'état sec et à l'état humide supérieur à 0,35 et, en particulier, un indice de friction moyen à l'état humide supérieur à l'indice de friction moyen à l'état sec.

11. Procédé de fabrication d'un article (1) de nettoyage et/ou de soin de la peau, comportant les étapes suivantes :

   - formation d'au moins une première couche externe (10a) dudit article (1) à partir d'une nappe de fibres absorbantes,
   - formation d'au moins une deuxième couche externe (10b) dudit article (1) à partir d'une nappe de fibres absorbantes,
   - insertion d'au moins une première série de fils (10c,10d,10e) entre lesdites première (10a) et deuxième (10b) couches externes,
   - assemblage desdites couches externes (10a, 10b) et de ladite série de fils (10c,10d,10e).

12. Procédé selon la revendication 11, **caractérisé en ce que** l'assemblage des couches externes (10a,10b) et de la série de fils (10c,10d,10e,10f) s'effectue au moyen d'une technique choisie parmi l'hydroliage, le collage et la thermofusion.

13. Procédé selon l'une quelconque des revendications 11 à 12, **caractérisé en ce qu'**on insère une deuxième série de fils (10f,10f1,10f2) entre les première et deuxième couches externes (10a,10b), les fils de chacune des première (10e) et deuxième (10f,10f1,10f2) séries étant sensiblement parallèles entre eux et formant un angle α avec les fils de l'autre série.

14. Procédé selon la revendication 13, **caractérisé en ce que** les fils de la première série (10e) sont déposés après et au-dessus des fils de la deuxième série (10f).

15. Procédé selon la revendication 14, **caractérisé en ce que** les fils de la deuxième série (10f1,10f2) sont déposés de manière à former avec les fils de la première série (10e) une structure semblable à une trame textile, les fils de la première série (10e) formant les fils de trame et les fils de la deuxième série (10f1,10f2) formant les fils de chaîne.

16. Procédé selon l'une quelconque des revendications 13 à 15, **caractérisé en ce que** l'angle α est sensiblement égal à 90°.

17. Procédé selon l'une quelconque des revendications 11 à 16, **caractérisé en ce que** les nappes de fibres (10a,10b) sont formées essentiellement à partir de fibres de coton hydrophiles.

18. Procédé selon la revendication 17, **caractérisé en ce que** les nappes de fibres (10a, 10b) comprennent de 70 à 100 % de fibres de coton et de 0 à 30 % de fibres artificielles, choisies notamment parmi les fibres de viscose, de fibres synthétiques, telles que des fibres de polyester, des fibres bicomposantes du type polyester/polyester, polypropylène/polypropylène ou polyester/polypropylène, ou leurs mélanges.

19. Procédé selon l'une quelconque des revendications 11 à 18, **caractérisé en ce que** les fils (10c,10d,10e,10f) sont fabriqués à partir d'un matériau choisi parmi les polymères d'origine naturelle, artificielle, synthétique, les matières métalliques et les matières minérales.

20. Procédé selon l'une quelconque des revendications 11 à 19, **caractérisé en ce que** les fils (10c, 10d, 10e, 10f) sont formés selon un procédé choisi parmi la filature, l'extrusion et le moulage.

**Claims**

1. Article (1) for skin cleansing and/or care, such as a pad for removing make-up, based on hydrophilic cotton fibres, designed to apply and/or remove liquid or semi-solid substances to or from the skin, comprising at least two outer layers (10a, 10b) made of an absorbent fibrous material bonded together and at least one series of yarns (10c, 10d, 10e, 10f) disposed between said outer layers (10a, 10b), **characterized in that** the thickness of at least one of the outer layers (10b) is less than the average diameter of the yarns so as to create a relief pattern at the surface of the article.

2. Article (1) according to Claim 1, **characterized in that** it comprises two series of yarns (10e, 10f) disposed between the outer layers (10a, 10b), the yarns of each of the series being substantially parallel to each other and forming an angle α with the yarns of the other series.

3. Article (1) according to Claim 2, **characterized in that** the two series of yarns are secured within a single woven grid (10c).

4. Article (1) according to either Claim 2 or 3, **characterized in that** the angle α is substantially equal to 90°.

5. Article (1) according to any one of the preceding claims, **characterized in that** the relief pattern possesses protuberances (5) with a height H of between 0.2 and 2.0 mm, and preferably between 0.3 and 0.5 mm.

6. Article (1) according to any one of the preceding claims, **characterized in that** the ratio Rh between the height H of the protuberances (5) of the relief pattern when the article is wet and the height H of the protuberances (5) of the relief pattern when the article is dry is greater than 0.7, and preferably greater than 1.

7. Article (1) for skin cleansing and/or care, such as a pad for removing make-up, based on hydrophilic cotton fibres, designed to apply and/or remove liquid or semi-solid substances to or from the skin, comprising at least two outer layers (10a, 10b) made of an absorbent fibrous material bonded together and at least one series of yarns (10c, 10d, 10e, 10f) disposed between said outer layers (10a, 10b), **characterized in that** said series of yarns creates a relief pattern at the surface of the article, said pattern possessing protuberances (5) with a height H and **in that** the ratio Rh between the height H of the protuberances (5) of the relief pattern when the article is wet and the height H of the protuberances (5) of the relief pattern when the article is dry is greater than 0.7 and preferably greater than 1.

8. Article (1) according to any one of the preceding claims, **characterized in that** the relief pattern defines a plurality of cavities designed to receive the cleansing and/or care product.

9. Article (1) according to any one of the preceding claims, **characterized in that** it possesses a tensile strength in the dry state of at least 35 N in the machine direction and at least 20 N in the cross direction and preferably greater than 50 N in the machine direction and cross direction according to the test method given in the description.

10. Article (1) according to any one of the preceding claims, **characterized in that** it possesses an average friction coefficient in the dry state and in the wet state greater than 0.35 and, in particular, an average friction coefficient in the wet state greater than the average friction coefficient in the dry state.

11. Method for producing an article (1) for skin cleansing and/or care, comprising the following steps:

   - formation of at least one first outer layer (10a) of said article (1) from a lap of absorbent fibres,
   - formation of at least one second outer layer (10b) of said article (1) from a lap of absorbent fibres,
   - insertion of at least one first series of yarns (10c, 10d, 10e) between said first (10a) and second (10b) outer layers,
   - assembling said outer layers (10a, 10b) and said series of yarns (10c, 10d, 10e).

12. Method according to Claim 11, **characterized in that** the outer layers (10a, 10b) and the series of yarns (10c, 10d, 10e, 10f) are assembled by means of a technique chosen from hydroentanglement, adhesive bonding and hot-melt bonding.

13. Method according to either of Claims 11 and 12, **characterized in that** a second series of yarns (10f, 10f1, 10f2) is inserted between the first and second outer layers (10a, 10b), the yarns of each of the first (10e) and second (10f, 10f1, 10f2) series being substantially parallel to each other and forming an angle $\alpha$ with the yarns of the other series.

14. Method according to Claim 13, **characterized in that** the yarns of the first series (10e) are positioned after and above the yarns of the second series (10f).

15. Method according to Claim 14, **characterized in that** the yarns of the second series (10f1, 10f2) are positioned so as to form, with the yarns of the first series (10e), a structure similar to a textile screen, the yarns of the first series (10e) forming the weft yarns and the yarns of the second series (10f1, 10f2) forming the warp yarns.

16. Method according to any one of Claims 13 to 15, **characterized in that** the angle $\alpha$ is substantially equal to 90°.

17. Method according to any one of Claims 11 to 16, **characterized in that** the laps of fibres (10a, 10b) are formed substantially from hydrophilic cotton fibres.

18. Method according to Claim 17, **characterized in that** the laps of fibres (10a, 10b) comprise 70 to 100% of cotton fibres and 0 to 30% of artificial fibres, chosen particularly from viscose fibres, synthetic fibres, such as polyester fibres, bicomponent fibres of the polyester/polyester type, polypropylene/polypropylene type or polyester/polypropylene type, or mixtures thereof.

19. Method according to any one of Claims 11 to 18 **characterized in that** the yarns (10c, 10d, 10e, 10f) are produced from a material chosen from polymers of natural, artificial or synthetic origin or metallic materials and mineral materials.

20. Method according to any one of Claims 11 to 19, **characterized in that** the yarns (10c, 10d, 10e, 10f) are formed by a method chosen from spinning, extrusion and moulding.

**Patentansprüche**

1. Artikel (1) zur Reinigung und/oder zur Pflege der Haut, wie ein Abschminkpad, auf der Basis von hydrophilen Baumwollfasern, der dazu bestimmt ist, flüssige oder halbflüssige Substanzen auf die Haut aufzutragen und/oder davon zu entfernen, der mindestens zwei miteinander verbundene Außenschichten (10a, 10b) aus absorbierendem Fasermaterial und mindestens eine Reihe von Fäden (10c, 10d, 10e, 10f) enthält, die zwischen den Außenschichten (10a, 10b) angeordnet ist, **dadurch gekennzeichnet, dass** die Dicke mindestens einer der Außenschichten (10b) geringer als der mittlere Durchmesser der Fäden ist, um ein Reliefmuster an der Oberfläche des Artikels zu erzeugen.

2. Artikel (1) nach Anspruch 1, **dadurch gekennzeichnet, dass** er zwei Reihen von Fäden (10e, 10f) enthält, die zwischen den Außenschichten (10a, 10b) angeordnet sind, wobei die Fäden jeder der Reihen im wesentlichen parallel zueinander sind und mit den Fäden der anderen Reihe einen Winkel α bilden.

3. Artikel (1) nach Anspruch 2, **dadurch gekennzeichnet, dass** die zwei Reihen von Fäden innerhalb eines einzigen Rastergitters (10c) fest verbunden sind.

4. Artikel (1) nach einem der Ansprüche 2 oder 3, **dadurch gekennzeichnet, dass** der Winkel α im Wesentlichen gleich 90° ist.

5. Artikel (1) nach einem der vorhergehenden Ansprüche, **dadurch gekennzeichnet, dass** das Reliefmuster Vorsprünge (5) einer Höhe H aufweist, die zwischen 0,2 und 2,0 mm, und vorzugsweise zwischen 0,3 und 0,5 mm liegt.

6. Artikel (1) nach einem der vorhergehenden Ansprüche, **dadurch gekennzeichnet, dass** das Verhältnis Rh zwischen der Höhe H der Vorsprünge (5) des Reliefmusters, wenn der Artikel feucht ist, und der Höhe H der Vorsprünge (5) des Reliefmusters, wenn der Artikel trocken ist, höher als 0,7 und vorzugsweise höher als 1 ist.

7. Artikel (1) zur Reinigung und/oder zur Pflege der Haut, wie ein Abschminkpad, auf der Basis von hydrophilen Baumwollfasern, der dazu bestimmt ist, flüssige oder halbflüssige Substanzen auf die Haut aufzutragen und/oder davon zu entfernen, der mindestens zwei miteinander verbundene Außenschichten (10a, 10b) aus absorbierendem Fasermaterial und mindestens eine Reihe von Fäden (10c, 10d, 10e, 10f) enthält, die zwischen den Außenschichten (10a, 10b) angeordnet ist, **dadurch gekennzeichnet, dass** die Reihe von Fäden ein Reliefmuster an der Oberfläche des Artikels erzeugt, wobei das Muster Vorsprünge (5) einer Höhe H besitzt, und dass das Verhältnis Rh zwischen der Höhe H der Vorsprünge (5) des Reliefmusters, wenn der Artikel feucht ist, und der Höhe H der Vorsprünge (5) des Reliefmusters, wenn der Artikel trocken ist, höher als 0,7 und vorzugsweise höher als 1 ist.

8. Artikel (1) nach einem der vorhergehenden Ansprüche, **dadurch gekennzeichnet, dass** das Reliefmuster eine Vielzahl von Hohlräumen definiert, die dazu bestimmt sind, das Reinigungs- und/oder Pflegeprodukt aufzunehmen.

9. Artikel (1) nach einem der vorhergehenden Ansprüche, **dadurch gekennzeichnet, dass** er eine Zugfestigkeit im Trockenzustand von mindestens 35 N in der Laufrichtung und von mindestens 20 N in der Querrichtung, und vorzugsweise höher als 50 N in der Laufrichtung und der Querrichtung besitzt, gemäß dem Testverfahren in der Beschreibung.

10. Artikel (1) nach einem der vorhergehenden Ansprüche, **dadurch gekennzeichnet, dass** er einen mittleren Reibungsindex im Trockenzustand und im Feuchtzustand höher als 0,35, und insbesondere einen mittleren Reibungsindex im Feuchtzustand höher als der mittlere Reibungsindex im Trockenzustand besitzt.

11. Verfahren zur Herstellung eines Artikels (1) zur Reinigung und/oder zur Pflege der Haut, das die folgenden Schritte aufweist:

    - Bilden mindestens einer ersten Außenschicht (10a) des Artikels (1) ausgehend von einer Lage von absorbierenden Fasern,
    - Bilden mindestens einer zweiten Außenschicht (10b) des Artikels (1) ausgehend von einer Lage von absorbierenden Fasern,
    - Einfügen mindestens einer ersten Reihe von Fäden (10c, 10d, 10e) zwischen die erste (10a) und die zweite (10b) Außenschicht,
    - Zusammenfügen der Außenschichten (10a, 10b) und der Reihe von Fäden (10c, 10d, 10e).

**12.** Verfahren nach Anspruch 11, **dadurch gekennzeichnet, dass** die Zusammenfügung der Außenschichten (10a, 10b) und der Reihe von Fäden (10c, 10d, 10e, 10f) mittels einer Technik erfolgt, die aus der Hydroverwirbelung, dem Kleben und dem Wärmeschmelzen ausgewählt wird.

**13.** Verfahren nach einem der Ansprüche 11 bis 12, **dadurch gekennzeichnet, dass** eine zweite Reihe von Fäden (10f, 10fl, 10f2) zwischen die erste und die zweite Außenschicht (10a, 10b) eingefügt wird, wobei die Fäden jeder der ersten (10e) und der zweiten (10f, 10fl, 10f2) Reihe im Wesentlichen parallel zueinander sind und einen Winkel $\alpha$ mit den Fäden der anderen Reihe bilden.

**14.** Verfahren nach Anspruch 13, **dadurch gekennzeichnet, dass** die Fäden der ersten Reihe (10e) nach und über den Fäden der zweiten Reihe (10f) aufgebracht werden.

**15.** Verfahren nach Anspruch 14, **dadurch gekennzeichnet, dass** die Fäden der zweiten Reihe (10f1, 10f2) so aufgebracht werden, dass sie mit den Fäden der ersten Reihe (10e) eine Struktur ähnlich einem Textilgitter bilden, wobei die Fäden der ersten Reihe (10e) die Schussfäden und die Fäden der zweiten Reihe (10f1, 10f2) die Kettfäden bilden.

**16.** Verfahren nach einem der Ansprüche 13 bis 15, **dadurch gekennzeichnet, dass** der Winkel $\alpha$ im Wesentlichen gleich 90° ist.

**17.** Verfahren nach einem der Ansprüche 11 bis 16, **dadurch gekennzeichnet, dass** die Faserlagen (10a, 10b) im Wesentlichen ausgehend von hydrophilen Baumwollfasern geformt werden.

**18.** Verfahren nach Anspruch 17, **dadurch gekennzeichnet, dass** die Faserlagen (10a, 10b) 70 bis 100 % Baumwollfasern und 0 bis 30 % Kunstfasern enthalten, die insbesondere ausgewählt werden aus den Viskosefasern, den Kunstfasern, wie Polyesterfasern, den Zweikomponentenfasern der Art Polyester/Polyester, Polypropylen/Polypropylen oder Polyester/Polypropylen, oder ihren Mischungen.

**19.** Verfahren nach einem der Ansprüche 11 bis 18, **dadurch gekennzeichnet, dass** die Fäden (10c, 10d, 10e, 10f) ausgehend von einem Material hergestellt werden, das ausgewählt wird aus den Polymeren natürlichen, künstlichen, synthetischen Ursprungs, den metallischen Materialien und den mineralischen Materialien.

**20.** Verfahren nach einem der Ansprüche 11 bis 19, **dadurch gekennzeichnet, dass** die Fäden (10c, 10d, 10e, 10f) gemäß einem Verfahren geformt werden, das aus dem Spinnen, dem Extrudieren und dem Formen ausgewählt wird.

**FIG.1**

**FIG.2A**

**FIG.2B**

**FIG.2C**

**FIG.2D**

FIG.3A

FIG.3B

11

10b        10e

10a        10f        12

**FIG.3C**

11

10b
10f1       10e

10a
10f2       12

**FIG.3D**

**FIG.4A**

**FIG.4B**

**FIG.4C**

**FIG.4D**

**FIG.4E**

**FIG.4F**

**FIG.4G**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0142548 A

- EP 0681621 A